# EUROPÄISCHE PATENTSCHRIFT

(11) **EP 0 326 069 B1**
(45) Veröffentlichungstag und Bekanntmachung des Hinweises auf die Patenterteilung: **22.06.1994**
(21) Anmeldenummer: 89101167.8
(22) Anmeldetag: 24.01.1989
(51) Int. Cl.: C05F 9/02, A01K 67/00

(54) **Verfahren und Hilfsvorrichtung zur Aufbereitung organischer Abfälle**
Process and apparatus for the treatment of organic wastes
Procédé et dispositif de traitement de déchets organiques

(30) Priorität: 25.01.1988 DE 3802011
(43) Veröffentlichungstag der Anmeldung: 02.08.1989
(73) Patentinhaber: Blessing, Gustav, D-79650 Schopfheim (DE)
(72) Erfinder: Blessing, Gustav, D-79650 Schopfheim (DE)
(74) Vertreter: Patent- und Rechtsanwaltssozietät, Schmitt, Maucher & Börjes

(56) Entgegenhaltungen:
- DE-A- 2 654 027
- DE-A- 3 015 164
- FR-A- 2 594 434
- LU-A- 85 188
- US-A- 3 654 903

## Beschreibung

Die Erfindung betrifft ein Verfahren zur Aufbereitung organischer Abfälle.

Täglich fallen in den Haushalten, Betrieben und Kommunen große Mengen Abfall an. Dieser wird bislang überwiegend zur Geländeauffüllung verwendet, ungeachtet dessen, daß dies eine Verschwendung noch verwertbarer oder wiederverwertbarer Materialien bedeutet und auch das Grundwasser empfindlich belasten kann. Mittlerweile stößt man zudem auch auf erhebliche Schwierigkeiten u.a. bei der Bevölkerung, neue Deponieplätze zu finden.

Da andererseits insbesondere organisches Material zu kostbar ist, um etwa nur verbrannt zu werden, ist man mancherorts bereits dazu übergegangen, solches Material getrennt zu sammeln und anschließend zu kompostieren.

So werden beispielsweise die größeren Stücke des getrennt gesammelten organischen Materials zunächst in einer Vor- und/oder einer Nachzerkleinerungsanlage zerkleinert, bevor sie über Förderbänder in einen Bunker den kleineren und zerkleinerten organischen Bestandteilen des Abfalls beigemischt werden. Mit Hilfe stumpfer Werkzeuge, etwa von Prallreißern, wird dieses Material anschließend noch weiter zerkleinert, aufgerissen und zerfasert. Beimischungen von Kalkstickstoff oder speziellen Algen sollen dabei als Kompostierungshilfen dienen. Dieses solchermaßen behandelte Abfallmaterial wird dann in Vorsiebanlagen von groben Gestankstoffen, wie beispielsweise Kunststoffen, Textilien, Holz, Dosen und Verpackungsstoffen befreit. Schließlich wird das so aufbereitete Substrat in Mieten von beispielsweise 30 m Länge und 4 m Höhe zur Verrottung aufgesetzt. Die Rottezeit beträgt etwa 6 bis 12 Monate. Während dieser Zeit sollte das Rohkompost-Material ständig umgesetzt und wieder locker und gleichmäßig aufgesetzt werden.

Dieses vorbekannte Kompostierungsverfahren hat jedoch wesentliche Nachteile. Es ist sehr kosten-, zeit- und arbeitsintensiv. Der Kompostierungsvorgang ist von Witterungseinflüssen abhängig. Insbesondere anaerobe Zersetzungsvorgänge führen zu einer in der Nähe von Wohngebieten kaum zumutbaren Geruchsbelästigung. Schließlich kann bei diesem Verfahren organisch belastetes, in Kläranlagen zu behandelndes Sickerwasser anfallen.

Man hat daher ein Kompostierungsverfahren geschaffen, bei dem der organische Abfall zunächst zerkleinert und entschrottet wird. Das Material wird dann über eine Förderbandanlage Rotte-Containern zugeführt, in denen die Kompostierung durch biothermische Trocknung in verschiedenen Temperatur- und Trocknungsphasen und begleitenden aeroben Prozessen erfolgt. Je nach Bedarf kann dieser Kompost anschließend beispielsweise einem Nachrottungsvorgang unterzogen, mit Zuschlagstoffen vermischt oder dem Ausgangsmaterial wieder zugeführt werden. Dieses Verfahren zeichnet sich durch eine wesentlich kürzere Kompostierungsdauer aus, hat aber seinerseits den Nachteil, daß das Endprodukt ohne weitere Nachbehandlung nur Rohkompost, d.h., ein noch weiter abbaubedürftiges Verrottungsprodukt bildet mit immer noch einem hohen Anteil unvollständig zersetzter organischer Substanz.

Aus der DE-OS 26 54 027 kennt man bereits einen Aufbereitungsbehälter, der ebenfalls als Komposter ausgebildet ist. Dieser vorbekannte Komposter besteht aus einem Behälter, dessen Behälterboden mit Abstand zur Standfläche des Komposters angeordnet ist. Um die Entleerung dieses Komposters möglichst einfach zu gestalten, ist dessen Behälterboden aus mehreren parallelen und drehbar gelagerten Rechen gebildet. Diese Rechen weisen Zinken auf, die in mehreren Zinkenreihen am Rechbalken gleichmäßig verteilt angeordnet sind. Durch Drehen der einzelnen Rechen können dessen Zinken jeweils das im Komposter verrottete Material durchfurchen und aufbrechen, so daß dieses Material anschließend in eine unterhalb des Behälters befindliche Gartenkarre oder ein anderes Auffangbehältnis fällt.

In der DE-OS 30 15 164 ist ein weiterer Komposter beschrieben, bei dem der Behälterboden aus mehreren Stäben gebildet ist, welche an beiden Enden frei zur Seite beweglich abgestützt sind und somit in der Bodenebene seitlich verschoben werden können. Durch die große brückenbildende Konsistenz des kompostierten Materials wird der Kompoststock von den entsprechend voneinander beabstandeten Stäben gehalten, ohne daß Kompost nach unten durchfällt. Durch ein Auseinanderrücken von zwei einander benachbarten Stäben und unter gleichzeitigem Seitwärtsschieben der ihnen auswärts benachbarten Stäbe kann der Abstand zwischen diesen beiden Stäben zur Kompostabgabe vorübergehend derart vergrößert werden, daß die Brücken des Kompostmaterials zusammenbrechen und dieses zwischen den auseinandergerückten Stäben nach unten durchfällt.

Auch bei diesem aus DE-OS 30 15 164 vorbekannten Komposter ist eine Entnahme des verrotteten Materials auf bequeme Weise möglich. Durch den Bodenabstand des Kompostbehälters kann außerdem der beim Kompostieren durch Befeuchtung und biologische Zersetzung entstehende Saft, der in konzentrierter Menge eine bewachsende Grundfläche unter dem Kompost schädigt, leicht aufgefangen werden.

Aus der FR-2 594 434 kennt man bereits eine Vorrichtung, die zur Aufbereitung organischer Abfälle mittels bodenbewohnender Gliederwürmer vorgesehen ist. Diese vorbekannte Vorrichtung besteht aus mehreren flachen und nach oben offenen Behältern, die übereinander und seitlich nebeneinander stapelbar sind. Die aneinander angrenzenden Schmalseiten sowie die Behälterböden dieser Behälter sind jeweils aus einem Gitter hergestellt, welches den Gliederwürmern ein Einwandern in den benachbarten Behälter erlaubt. Durch ein Umschichten der untereinander austauschbaren Behälter dieser vorbekannten Vorrichtung kann zur Aufbereitung ständig neues Material zugeführt werden, welches die Würmer zu einem Überwechseln in den Behälter mit frischerem organischen Abfall veranlaßt. Die Würmer setzen diesen Abfall in den einzelnen Behältern in Wurmhumus um, der sich durch seine hohe Qualität und seine große Homogenität auszeichnet.

Ein Aufbereitungsbehälter zur Aufbereitung organischer Abfälle mittels bodenbewohnender Gliederwürmer ist auch aus der LU-85 188 bekannt. Dieser vorbekannte Aufbereitungsbehälter weist zwei Behälterteile auf, zwischen denen ein Gitterrost als Trennboden vorgesehen ist. Der Gitterrost kann aus dem Behälterinneren des Aufbereitungsbehälters durch einen Längsspalt geschoben werden, welcher zwischen den beiden Behälterteilen vorgesehen ist. Das mit Würmern vesehene Abfallmaterial wird bei diesem vorbekannten Aufbereitungsbehälter gemäß LU-85 188 zunächst in das durch den Boden verschlossene untere Behälterteil eingebracht. Sobald die Würmer das im unteren Behälterteil enthaltene Material in Wurmhumus umgesetzt haben, wird oberhalb des Gitterrostes weiteres Abfallmaterial in den oberen Behälterteil eingesetzt. Dabei können die Würmer vom unteren Behälterteil durch den Gitterrost in das obere Behälterteil überwechseln. Sobald dies geschehen ist, wird der Boden des unteren Behälterteiles geöffnet und der Wurmhumus von dort entleert. Nach Verschließen des unteren Behälterteiles mittels des Behälterbodens kann der Gitterrost aus dem Behälterinneren des Aufbereitungsbehälters gezogen werden, so daß das zunächst noch auf dem Gitterrost lastende, die Würmer enthaltende Abfallmaterial in den unteren Behälterteil fällt.

Es besteht nun die Aufgabe, ein Verfahren zu entwickeln, das auch in größeren Anlagen die Aufbereitung und Umwandlung organischer Abfälle in hochwertigen Humus erlaubt, dabei kaum mit Geruchsbelästigungen verbunden ist und bei dem auch kein organisch belastetes und daher notwendig zu klärendes Sickerwasser entsteht.

Die erfindungsgemäße Lösung dieser Aufgabe besteht in den Merkmalen des Anspruches 1.

Das erfindungsgemäße Verfahren ist in einem Aufbereitungsbehälter durchzuführen, der eine oben befindliche Eingabeöffnung sowie einen Trennboden zum Abtrennen einer oberen Schicht von einer unteren Schicht aufweist, wobei der Trennboden aus mehreren, parallel nebeneinander angeordneten und zum Abfräsen der unteren Schicht in ihrer Längsrichtung drehbar gelagerten Fräsern besteht, die als lamellenförmige Kreuz-Trennelemente ausgebildet sind. In diesem Aufbereitungsbehälter werden die Abfälle in einem sich wiederholenden Prozeß etwa flach aufgeschichtet. Mittels der über eine Drehmechanik gemeinsam bewegbaren Kreuz-Trennelemente kann nun in Zeitabständen die unterhalb der die Würmer zumindest überwiegend enthaltene Schicht befindliche, in Wurmhumus verarbeitete untere Schicht abgefräst werden. Dabei kann etwa durch die Wahl eines bestimmten Zeitpunktes sichergestellt werden, daß die Würmer sich weitgehend in der oberen Schicht aufhalten.

Im Gegensatz zu einem Schieber, der bei beispielsweise nur grob zerkleinertem Abfallmaterial mit nicht unerheblichem Kraftaufwand durch das Substrat geschoben werden muß, kann bei dem Trennboden des für das erfindungsgemäße Verfahren vorgesehenen Aufbereitungsbehälters durch eine bloße Drehung der Kreuz-Trennelemente um 45° bzw. 90° eine Trennung des Materials in zwei Schichten und ein Abfräsen der fertigen Wurmhumusschicht erreicht werden. Dabei muß - im Vergleich zu einem Trennschieber - nur wenig Material nach oben und unten verdrängt werden.

In der Schließstellung bilden die Breitseiten der lamellenförmigen Trennelemente praktisch eine durchgehende Trennebene. Werden die Kreuz-Trennelemente um 45° in die Öffnungsstellung gekippt, so stehen in Durchfallrichtung lediglich die Schmalseiten der Trennelemente entgegen und das oberhalb des Trennbodens befindliche Schichtmaterial kann leicht nach unten fallen. Eine Drehmechanik oder ein Kettenantrieb, welche die Trennelemente gemeinsam von der Öffnungs- in die Schließstellung bringen, erlauben dabei einen schnellen und unkomplizierten Trenn- oder Fräsvorgang. Dabei kann eine einfache und vorteilhafte Drehmechanik so ausgebildet sein, daß die Kreuz-Trennelemente vorzugsweise an einem außenliegenden Ende jeweils Kurbelarme oder Ketten aufweisen, die mit einer Schubstange oder Kettenrädern verbunden sind, wobei ein Verstellantrieb an dieser Schubstange angreift.

Bei dem erfindungsgemäßen Verfahren wird den bodenbewohnenden Gliederwürmern im Aufbereitungsbehälter ständig weiteres organisches Material zugeführt. Solche Gliederwürmer, zu denen der einheimische sogenannte Regenwurm zählt, aber auch der hier bevorzugt eingesetzte kalifornische Rotwurm (Eisenea foetida), ernähren sich von organischen Abfällen. Diese Abfallstoffe werden von den Würmern in sogenannten Wurmhumus umgewandelt, der als wertvoller und begehrter Dünger gilt. Dieser Wurmhumus vermag die physikalische Bodenstruktur zu verbessern, hat einen hohen Nährstoffgehalt, setzt in vorteilhafter Weise diese Nährstoffe langsamer frei als Mineraldünger und erhöht dadurch insgesamt die Wiederstandskraft der Pflanzen.

Die Würmer können in den Aufbereitungsbehälter unter für sie idealen oder zumindest doch guten Bedingungen gehalten werden. Dabei erlaubt das erfindungsgemäße Verfahren deren rationellen und kostengünstigen Einsatz mit nur wenig Personal. Denn den Aufbereitungsbehältern kann in Zeitintervallen ständig neues Material zugeführt und durch das Unterteilen in eine wurmhaltige und eine verarbeitete Schicht ebenfalls in Zeitabständen der Wurmhumus laufend entnommen werden. Dabei wird ein Verarbeitungsverfahren geschaffen, bei welchem insoweit weitgehend auf ein manuelles Zutun verzichtet werden kann. Da die ständig aufgetragenen Abfallschichten vergleichsweise eine geringe Höhe haben und zudem nicht übermäßig feucht sind, werden insbesondere anaerobe Zersetzungsvorgänge und dergleichen Geruchsbelästigungen weitgehend vermieden. Durch die vergleichsweise kurze Durchlaufzeit der Abfallstoffe bis zu ihrer Umsetzung ist auch der Platzbedarf für ein solches Verfahren vergleichsweise gering. Lange, dem Regen ausgesetzte Mieten erübrigen sich ebenso, wie die bei vorbekannten Verfahren durch belastetes Sickerwasser auftretenden Probleme.

Es läßt sich kaum vermeiden, daß insbesondere auf einem als Sieb- oder Lochblech ausgebildeten unteren Behälterboden größere Materialreste, aber auch Steine oder dergleichen im Laufe der Zeit liegenbleiben. Es ist daher vorteilhaft, wenn der Behälterboden zu einer Schüttöffnung hin schräg nach unten geneigt ist, damit über diese Schüttöffnung solche Gegenstände aus dem Verarbeitungsprozeß entfernt werden können.

Um den Wurmhumus mehrerer solcher Aufschichtungs-Durchläufe rationell zu sammeln, ist zweckmäßigerweise unterhalb des Behälterbodens ein Auffangbehälter vorgesehen.

Zwar bevorzugen die Würmer einen gewissen Feuchtigkeitsgehalt im Boden; der Boden darf aber auch nicht zu feucht sein, weil andernfalls die Wurmhumus-Produktion der Würmer wieder zurückgeht. Zweckmäßigerweise ist daher die Eingabeöffnung durch einen Deckel oder mehrere Deckelteile regendicht verschließbar. Dies hat darüber hinaus den Vorteil, daß eine zu starke Durchfeuchtung des Abfallmaterials und eine Geruchsbelästigung der Umgebung weitgehend vermieden wird.

Um den Würmern auch von ihren Wärmeanforderungen her optimale Lebensbedingungen zu bieten, ist es zweckmäßig, wenn der Deckel oder die Deckelteile an ihrer Oberseite Solarzellen aufweisen, die mit Wärmetauschern im Behälterinneren in Verbindung stehen. Aus dem gleichen Grunde ist es auch vorteilhaft, wenn zumindest die Seitenwände des Aufbereitungsbehälters aus einem wärmeisolierenden Material bestehen und/oder von einer Wärmeisolierung umgeben sind. Diese Ausführungsformen gewährleisten ein weitgehend von den Außentemperaturen, den Jahreszeiten und den Witterungsbedingungen unabhängiges Aufbereitungsverfahren.

Zur Durchführung des erfindungsgemäßen Verfahrens wird eine Hilfsvorrichtung mit zwei Arbeitsstationen bevorzugt, wobei eine erste Arbeitsstation einen Häcksler oder dergleichen Zerkleinerer und eine zweite Arbeitsstation zumindest einen, das zerkleinerte Abfallmaterial aufbereitenden Aufbereitungsbehälter mit jeweils darunter angeordnetem Auffangbehälter aufweist und die Arbeitsstationen über zumindest eine Fördereinrichtung miteinander verbunden sind. Eine solche Hilfsvorrichtung erlaubt eine sehr rationelle Durchführung des Aufbereitungsprozesses mit vergleichsweise geringem Personal-, Arbeits- und Kostenaufwand. Dabei kann der ersten Arbeitsstation, in der das organische Abfallmaterial zerkleinert wird, ein Sortiervorgang vorgeschaltet werden, wenn aus dem Abfall für die Würmer etwa nur schwer verdauliches organisches Abfallmaterial, wie beispielsweise Rohmist, ausgesondert werden muß.

Nachstehend wird die Erfindung anhand eines vorteilhaften Ausführungsbeispieles in Verbindung mit den Zeichnungen noch näher erläutert.

Es zeigen:
- Fig. 1: eine Hilfsvorrichtung zur Aufbereitung organischer Abfälle in einer Gesamtansicht,
- Fig. 2: einen Aufbereitungsbehälter mit einem aus Kreuz-Trennlamellen oder Fräsern gebildeten Trennboden,
- Fig. 3: den Verfahrensablauf des Aufbereitungsverfahrens im Aufbereitungsbehälter in neun Einzelschritten (Fig. 3a bis Fig. 3i) und
- Fig. 4: einen Aufbereitungsbehälter während des Umsetzens von einem Auffangbehälter zum anderen.

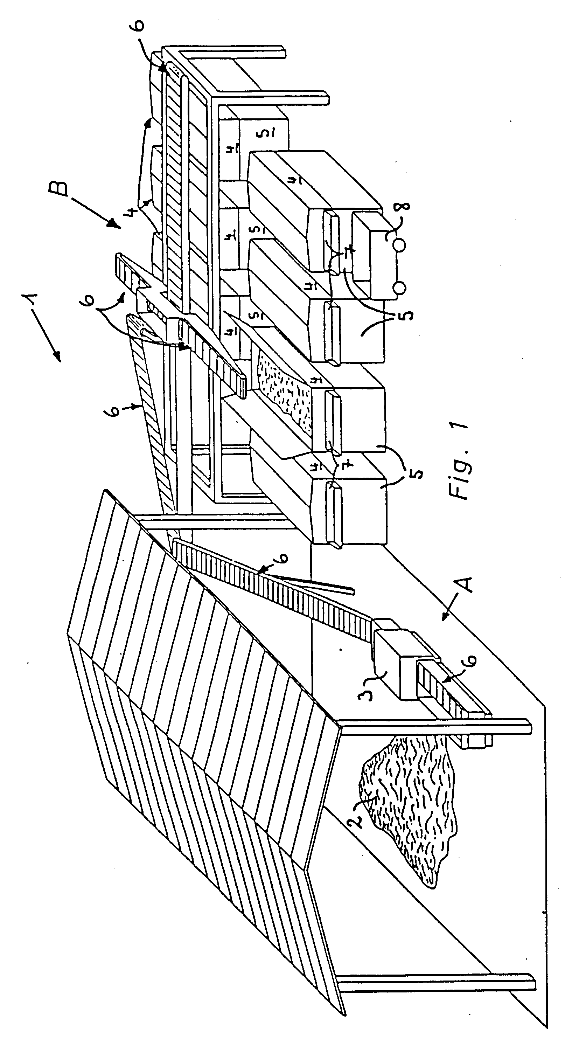

Figur 1 zeigt eine im Ganzen mit 1 bezeichnete Hilfsvorrichtung zur Aufbereitung organischer Abfälle 2 in einer Gesamtansicht. Die Hilfsvorrichtung 1 hat zwei Arbeitsstationen A und B, wobei die erste Arbeitsstation A einen Häcksler 3 und die zweite Arbeitsstation B zumindest sieben in Figur 1 sichtbare, das zerkleinerte Abfallmaterial aufbereitende Aufbereitungsbehälter 4, jeweils mit einem darunter angeordneten Auffangbehälter 5 aufweist.

Der getrennt gesammelte und in Figur 1 zu einem großen Haufen aufgehäufte organische Abfall 2 wird zunächst über eine als Förderband 6 ausgebildete Fördereinrichtung dem Häcksler 3 zugeführt und dort zerkleinert. Über ein weiteres Förderband 6 verlassen die zerkleinerten organischen Abfälle 2 den überdachten Bereich der Arbeitsstation A und werden einem der Aufbereitungsbehälter 4 zugeführt. Dort werden die zerkleinerten organischen Abfälle 2 mit Hilfe von bodenbewohnenden Gliederwürmern, vorzugsweise mit Hilfe des kalifornischen Rotwurms, größtenteils in krümeligen Wurmhumus aufbereitet. Sind die organischen Abfälle 2 im Aufbereitungsbehälter 4 von den Würmern weitgehend umgesetzt worden, so wird der krümelige Wurmhumus in den Aufbereitungsbehältern 4 der Arbeitsstation B durchgesiebt und fällt nach unten in den darunter angeordneten Auffangbehälter 5.

Da nie ganz ausgeschlossen werden kann, daß in die Abfälle 2 auch Steine oder dergleichen feste Bestandteile geraten, weisen die Aufbereitungsbehälter 4 Schüttöffnungen 7 auf. Über die Schüttöffnungen 7 können Steine oder dergleichen etwa beim Sieben aus dem Aufbereitungsbehälter 4 nach außen und beispielsweise in einen Schüttmaterial-Wagen 8 fallen.

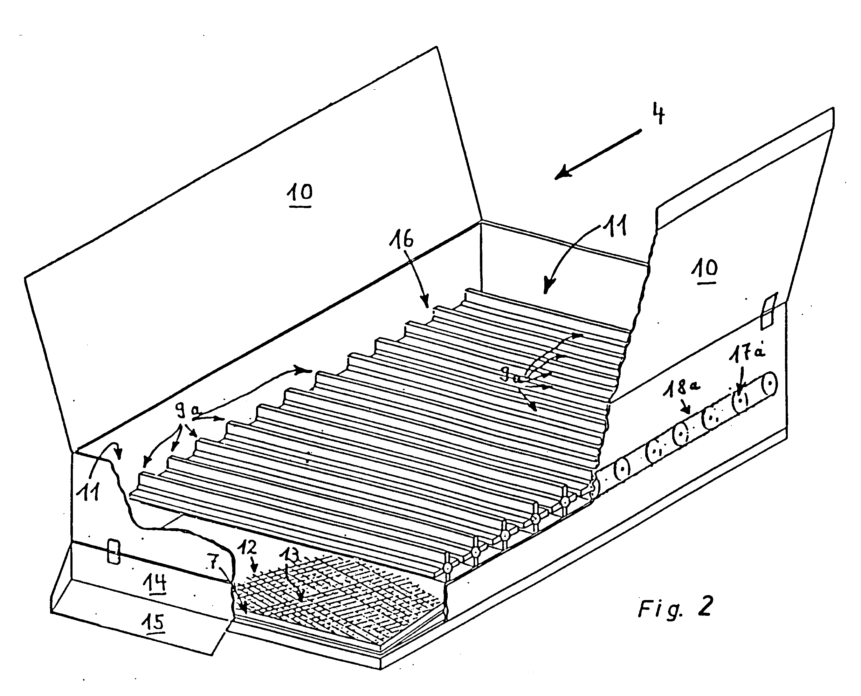

Figur 2 zeigt einen Aufbereitungsbehälter 4'' mit in Schließstellung eine Trennebene bildenden, kreuzlamellenförmigen Kreuz-Trennelementen 9a teilweise aufgebrochen und in einer perspektivischen Seitenansicht dargestellt. Der Aufbereitungsbehälter 4'' weist eine durch zwei Deckelteile 10 regendicht verschließbare Eingabeöffnung 11 auf. Durch die Eingabeöffnung 11 wird der zerkleinerte organische Abfall in das Behälterinnere eingebracht.

In Figur 2 ist der untere Behälterboden 12 als Sieb 13 ausgebildet. Die Schüttöffnung 7 wird durch eine Klappe 14 verschlossen und geht nach außen in einen Trichter 15 über. Zwischen Eingabeöffnung 11 und unterem Behälterboden 12 ist ein in Öffnungs- und Schließstellung bringbarer Trennboden oder Fräser 16 angeordnet.

In Figur 2 besteht der Trennboden 16 im wesentlichen aus den parallel nebeneinander angeordneten, in Längsrichtung drehbar gelagerten, lamellenförmigen Kreuz-Trennelementen 9a. Dabei sind die vorderen Trennelemente 9a in Figur 2 nur teilweise dargestellt, um den Blick auf den unteren Behälterboden 12 freizugeben. Die Trennelemente 9a weisen an einem außenliegenden Ende Kettenräder 17a auf, die mit einer Kette 18a verbunden sind. In Figur 2 befinden sich die Trennelemente 9a in ihrer Schließstellung. Durch einen Verstellantrieb über die Kettenräder 17a und die Kette 18a können die drehbar gelagerten Trennelemente 9a gedreht werden.

Der in Figur 2 nicht abgebildete organische Abfall 2 wird über die Eingabeöffnung 11 in einer ersten Schicht zunächst auf den oberen Behälterboden 16 aufgebracht. Die Schichthöhe kann beispielsweise 10 cm betragen und ist so bemessen, daß die Würmer optimal arbeiten können. Auf diese Schicht aus mit Würmern durchsetzten Abfällen 2 wird, wenn sich die Würmer von unten nach oben weitgehend durchgefressen haben, nach einiger Zeit - in der Regel nach einigen Tagen - eine weitere Schicht aufgetragen. Dieses Aufschichten des im Häcksler 3 zerkleinerten organischen Abfalls 2 erfolgt, bis der Aufbereitungsbehälter 4'' etwa gefüllt ist. Anschließend erfolgt eine Trennung oder Abfräsung der Gesamtschicht durch die in Öffnungsstellung gebrachten, lamellenartigen Kreuz-Trennelemente 9a. Dieser Zeitpunkt wird so gewählt, daß sich die Würmer überwiegend in dem oberhalb des Trennbodens 16 liegenden Bereich befinden. Dagegen wird der unterhalb des Trennbodens 16 liegende Bereich aus dem Behälterinneren des Aufbereitungsbehälters 4'' in den in Figur 2 nicht abgebildeten - Auffangbehälter 5 befördert.

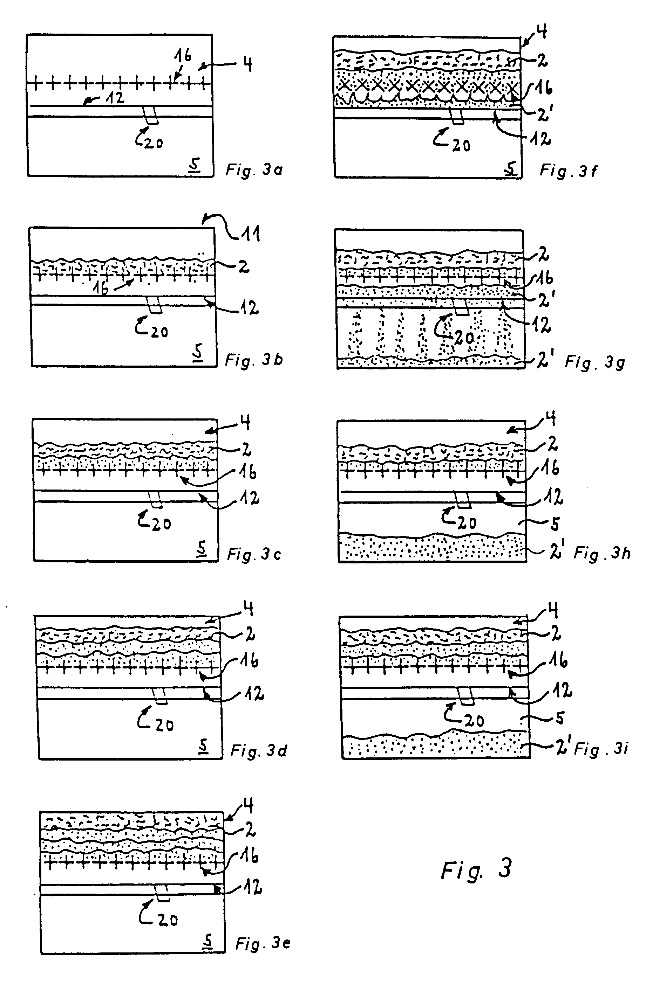

Dazu weist der als Sieb 13 ausgebildete untere Behälterboden 12 einen Exzentervibrator 20 (vgl. Figur 3) als Antrieb zum Durchführen von Siebbewegungen auf. Durch die Siebbewegungen werden gleichzeitig größere, unerwünschte Bestandteile aus dem Wurmhumus-Material ausgesiebt und über die Schüttöffnung 7 nach dem Behälterboden 12 nach außen befördert. Nach Beendigung dieses Siebvorganges kann durch Öffnen des Trennbodens 16 das in Schließstellung auf diesem liegende Schichtmaterial nach unten auf den unteren Behälterboden 12 fallen und sich der Schicht- und Aufbereitungsvorgang wiederholen.

Figur 3 soll den Verfahrensablauf des Aufbereitungsprozesses im Aufbereitungsbehälter 4'' veranschaulichen.

Figur 3a zeigt, stark schematisiert, den Aufbereitungsbehälter 4 sowie den darunter angeordneten Auffangbehälter 5 in einer seitlichen Schnittdarstellung. Im oberen Bereich des Aufbereitungsbehälters 4 ist als Kreuz der Trennboden 16 in seiner Schließstellung zu sehen. Der untere Behälterboden 12 dagegen ist darunter als durchgezogene Linie dargestellt; er ist zu einer Figur 3a nicht dargestellten Schüttöffnung 7 hin schräg nach unten geneigt.

Figur 3a, b zeigt den zerkleinerten organischen Abfall 2, wie er durch die Eingabeöffnung 11 auf den in Schließstellung befindlichen Trennboden 16 in einer ersten Schicht aufgetragen wird. Die in der ersten Schicht extra zugesetzten Würmer fressen sich von unten nach oben durch den Abfall 2 hindurch und arbeiten diesen in einen wertvollen, krümeligen Wurmhumus um. Haben sich die Würmer weitgehend durch die erste Schicht hindurchgefressen, so wird eine weitere Schicht von organischen Abfällen 2 aufgebracht. Dies wird, Schicht um Schicht, mehrmals wiederholt, bis das im Aufbereitungsbehälter 4 befindliche Substrat eine bestimmte Füllhöhe erreicht hat. Dieser Vorgang ist in den Figuren 3c bis 3e dargestellt.

Ist der Aufbereitungsbehälter 4 nahezu gefüllt, so wird der Trennboden 16 von der Schließ- in eine Öffnungsstellung gebracht. Dadurch wird das im Aufbereitungsbehälter befindliche Substrat in zwei Schichten unterteilt oder abgefräst, und zwar in eine oberhalb und eine unterhalb des Trennbodens 16 befindliche Schicht. Die oberhalb des Trennbodens 16 befindliche Schicht besteht noch weitgehend aus nicht aufbereiteten, zerkleinerten organischen Abfällen 2. Dementsprechend finden sich in dieser Schicht auch überwiegend die Würmer. Die unterhalb des Trennbodens 16 liegende Schicht dagegen besteht praktisch nur aus Wurmhumus 2' und ist weitgehend wurmfrei. Dieser Verfahrensabschnitt ist in Figur 3f dargestellt.

Figur 3g zeigt, wie nach Umschalten des Trennbodens 16 in seiner Schließstellung der untere Behälterboden 12 geöffnet wird. Dies erfolgt beispielsweise dadurch, daß der als Sieb ausgebildete Behälterboden 12 in Siebbewegungen versetzt wird. Figur 3g zeigt, wie ein in Siebbewegungen versetzter unterer Behälterboden 12 den Wurmhumus 2' nach unten in den Auffangbehälter 5 fallen läßt.

In Figur 3h befindet sich der Wurmhumus 2' aus dem Aufbereitungsbehälter 4 vollständig im Auffangbehälter 5. Da in diesem Verfahrensabschnitt der Trennboden 16 in seiner Schließstellung bleibt, kann die die Würmer enthaltene obere Schicht auf dem Trennboden 16 weitere Schichten aufnehmen.

Figur 3i zeigt, wie - ähnlich wie in Figur 3c - wieder Schicht um Schicht organischer Abfall 2 auf den Trennboden 16 aufgetragen wird.

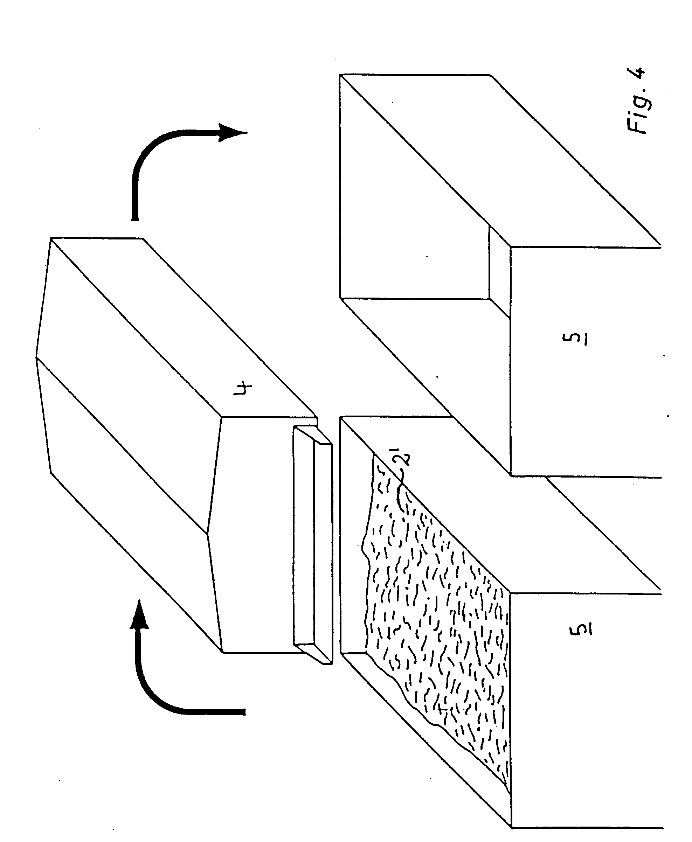

Figur 4 schließlich zeigt einen Aufbereitungsbehälter 4 während des Umsetzens von einem Auffangbehälter 5 zum anderen. Nach mehreren Durchgängen des Aufbereitungsverfahrens im Aufbereitungsbehälter 4 füllt sich auch der Aufnahmebehälter 5. Hat dieser nahezu sein Fassungsvermögen erreicht, so wird er gegen einen anderen Auffangbehälter 5 ausgetauscht. Dies erfolgt entweder dadurch, daß der Aufbereitungsbehälter 4 umgesetzt oder der unter ihm angeordnete Auffangbehälter 5 durch einen anderen ersetzt wird.

## Patentansprüche

1. Verfahren zur Aufbereitung organischer Abfälle in einem Aufbereitungsbehälter, der eine oben befindliche Eingabeöffnung sowie einen Trennboden zum Abtrennen einer oberen Schicht von einer unteren Schicht aufweist, wobei der Trennboden aus mehreren, parallel nebeneinander angeordneten und zum Abfräsen der unteren Schicht in ihrer Längsrichtung drehbar gelagerten Fräsern besteht, wobei die Abfälle mittels bodenbewohnender Gliederwürmer in einem Aufbereitungsbehälter (4, 4'') aufbereitet werden, dessen Fräser als lamellenförmige Kreuz-Trennelemente ausgebildet sind, wobei die Abfälle in einem sich wiederholenden Prozeß in Zeitabständen etwa flach aufgeschichtet werden und wobei die unterhalb der die Würmer zumindest überwiegend enthaltene Schicht befindliche, in Wurmhumus (2'') verarbeitete untere Schicht in Zeitabständen mittels der über eine Drehmechanik gemeinsam bewegbaren Kreuz-Trennelemente (9a) abgefräst wird.

## Claims

1. Process for preparing organic wastes in a dressing container having an input opening at the top and also a separating plane/bottom for cutting off a superior layer from an inferior layer, the separating plane/bottom consisting of several turnably seated cutters for cutting off the inferior layer in its longitudinal direction and which cutters being arranged parallel beside each other, with the wastes being dressed in a dressing container (4, 4'') by means of articulate worms living in the soil, the cutter of the dressing container being formed as lamellar cross separating elements, with the wastes being piled up about flat in periods of times in a repeated procedure and with the inferior layer transformed into worm humus (2'') situated below the layer containing the worms at least predominatly is milled off in periodic intervals by means of the cross separating elements (9a) jointly moveable due to a turnable mechanism.

## Revendications

1. Procédé pour le traitement de déchets organiques dans un conteneur de traitement, qui a un orifice d'entrée située en haut ainsi qu'un fond séparateur pour séparer une couche supérieure d'une couche inférieure, le fond séparateur consistant de plusieurs fraises logées tournables dans leur sens longitudinal et arrangées parallèlement les unes à côté de les autres, et les déchets étant traités dans un conteneur de traitement (4, 4'') moyennant des vers articulés vivant dans le sol, les fraises de ce conteneur étant formées comme éléments séparateurs cruciformes lamellaires avec les déchets étant empilés par couches dans un processus se répétant en intervalles périodiques environ platement et avec la couche inférieure transformée en humus de vers (2'') située au-dessous de la couche contenant au moins pour la plupart les vers étant fraisée en intervalles de temps moyennant des éléments séparateurs cruciformes (9a) qui peuvent être mûs ensemble moyennant un mécanisme tournant.
